# EUROPEAN PATENT SPECIFICATION

(11) **EP 3 310 436 B1**
(45) Date of publication and mention of the grant of the patent: **31.10.2018**
(21) Application number: 16731986.2
(22) Date of filing: 22.06.2016
(51) Int. Cl.: A61N 5/06, A61B 10/00, A61B 5/145

(54) **PHOTOTHERAPY APPARATUS WITH INTEGRATED URINE COLLECTOR AND SENSOR ENABLING REDUCTION OF SIDE-EFFECTS**
LICHTTHERAPIEVORRICHTUNG MIT INTEGRIERTEM URINKOLLEKTOR UND SENSOR ZUR REDUKTION VON NEBENWIRKUNGEN
APPAREIL DE PHOTOTHÉRAPIE AVEC COLLECTEUR D'URINE INTÉGRÉE ET CAPTEUR PERMETTANT LA RÉDUCTION DES EFFETS SECONDAIRES

(30) Priority: 22.06.2015 US 201562182661 P
(43) Date of publication of application: 25.04.2018
(73) Proprietor: Koninklijke Philips N.V., 5656 AE Eindhoven (NL)
(72) Inventor: VERVER-KLOMPENHOUWER, Muriëlle Maria, 5656 AE Eindhoven (NL); ULMAN, Shrutin, 5656 AE Eindhoven (NL); VAN ABEELEN, Frank Anton, 5656 AE Eindhoven (NL); PAULUSSEN, Elvira Johanna Maria, 5656 AE Eindhoven (NL)
(74) Representative: Kapoor, Pavan Puneet
(86) International application number: PCT/IB2016/053694
(87) International publication number: WO 2016/207799

(56) References cited:
- EP-A1- 0 747 002
- WO-A2-00/49948
- WO-A2-2007/091188
- US-A1- 2012 238 835

## Description

### FIELD

The following relates generally to measuring and treating hyperbilirubinemia and related conditions. It finds particular application in conjunction with a phototherapy apparatus for treating hyperbilirubinemia in a neonate, and is described with particular reference thereto. However, it is to be understood that it also finds application in other usage scenarios and is not necessarily limited to the aforementioned application.

### BACKGROUND

Neonatal hyperbilirubinemia is a condition for which neonatal jaundice is a common symptom. The condition results from insufficient removal of bilirubin from the blood. Bilirubin is a waste product produced during the breakdown of red blood cells, and is ordinarily removed by the liver. In the case of a fetus, the mother's liver supplements or performs this task, and neonatal hyperbilirubinemia arises when the neonate's liver is delayed in taking up this task. Neonatal hyperbilirubinemia is commonly present for the first 4-5 days after birth, but can last longer in premature infants, and becomes a serious problem if the total (blood) serum bilirubin (TSB) becomes elevated to the point where it builds up to toxic levels, especially in brain tissue (a condition known as kernicterus).

Phototherapy is a common treatment for neonatal hyperbilirubinemia. Light preferably in the 460-490 nm wavelength range illuminating the skin operates to convert the bilirubin to a form that is less lipophilic and hydrophobic and therefore can be excreted via the urine (and feces) without processing by the liver. Various commercial phototherapy devices are available. As another example, a phototherapy blanket in which LEDs are embedded in an infant-conformal blanket is disclosed in Asvadi et al., Int'l. Pub. WO 2007/091188 A2.

The following provides new and improved methods and systems which overcome the above-referenced problems and others.

### BRIEF SUMMARY

Phototherapy for treating hyperbilirubinemia employs illumination in the blue end of the visible spectrum, and is generally considered to have negligible detrimental side effects. Accordingly, monitoring of hyperbilirubinemia phototherapy has typically entailed measuring TSB level 4-6 hours after commencement of treatment to determine efficacy and thereafter every 12 hours until the TSB level reaches an acceptably low level to terminate the phototherapy. Such TSB measurements are commonly measured via drawn blood samples.

However, some possible detrimental side effects of phototherapy have been recognized, such as the possibility of thermal effects, water loss, electrolyte disturbance, bronze baby syndrome, circadian rhythm disorder, and potential longer-term side effects such as melanocytic nevi, skin cancer, allergic diseases, patent ductus arteriosus, and so forth. Phototherapy may also lead to increased oxidative stress due to production of reactive oxygen species and the reduction of bilirubin (which is a potent antioxidant).

To mitigate these side effects, hyperbilirubinemia phototherapy apparatuses and methods are disclosed herein which provide more frequent, and noninvasive, monitoring of one or more target biomarkers in urine. In one illustrative embodiment, a phototherapy blanket is provided with a urine reservoir (e.g. a diaper or other urine-absorbent area) and one or more built-in biomarker sensors are arranged to detect target biomarkers indicative of undesirable side effects and/or characterizing the liver function of the neonate with respect to bilirubin elimination (and hence indicative of the need to continue the phototherapy). Since a neonate typically urinates every 4-6 hours followed by diaper change-out, this approach provides noninvasive monitoring, and provides monitoring at a higher frequency than conventional TSB blood testing every 12 hours without creating an extra burden for the neonate and/or the caregiver.

In some embodiments, a target urine biomarker class is isoprostanes, which provide a measure of lipid peroxidation related to phototherapy breakdown of bilirubin. This can be monitored by an immunoassay that exhibits an observable color change, which can be detected visually by the caregiver or by optical sensor(s). Another suitable biomarker is urobilin, whose presence in urine is indicative of normal bilirubin removal by the liver. Urobilin colorizes urine and can be automatically detected using suitable optical sensor(s).

In accordance with one illustrative example, a hyperbilirubinemia phototherapy apparatus includes a phototherapy device including illuminators arranged to illuminate a neonate with phototherapy illumination effective to treat hyperbilirubinemia. A urine collector is configured to collect urine excreted by a neonate while being illuminated with phototherapy illumination by the phototherapy device. A sensing device is secured to at least one of the phototherapy device and the urine collector. The sensing device is configured to output a measurement of a target biomarker in urine collected by the urine collector.

In accordance with another illustrative example, a hyperbilirubinemia phototherapy apparatus includes a phototherapy device with illuminators arranged to illuminate a neonate with phototherapy illumination effective to treat hyperbilirubinemia. A sensing device is secured to the phototherapy device and configured to output a measurement of a target biomarker in urine excreted by a neonate receiving phototherapy illumination from the phototherapy device.

In accordance with another illustrative example, a hyperbilirubinemia phototherapy method includes administering hyperbilirubinemia phototherapy to a subject using a hyperbilirubinemia phototherapy device. During the administration of hyperbilirubinemia phototherapy, a sensing device is used to measure a target biomarker in urine excreted by the subject.

One advantage resides in providing more timely monitoring of the need of continued phototherapy and/or side effects of phototherapy treatment of hyperbilirubinemia. Another advantage resides in providing a phototherapy device with a data-driven therapy setting recommendation component.

Still further advantages of the present invention, which is defined in claim 1, will be appreciated to those of ordinary skill in the art upon reading and understand the following detailed description. It will be appreciated that a given embodiment may provide none, one, two, or more of these advantages.

### BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.
FIGURE 1 diagrammatically illustrates a hyperbilirubinemia phototherapy apparatus for administering hyperbilirubinemia phototherapy to a neonate.
FIGURE 2 diagrammatically illustrates a suitable embodiment of the sensing device of the hyperbilirubinemia phototherapy apparatus of FIGURE 1.
FIGURE 3 presents an exemplary flow chart of a hyperbilirubinemia phototherapy method suitably performed using the apparatus of FIGURE 1.

### DETAILED DESCRIPTION

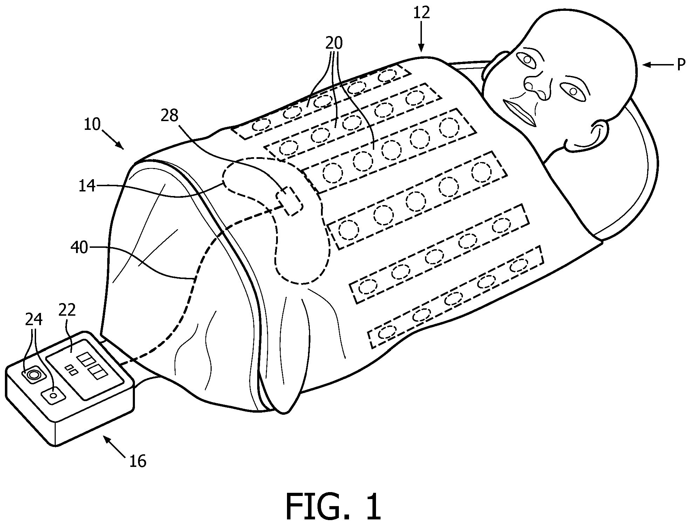

With reference to FIGURE 1, a hyperbilirubinemia phototherapy apparatus **10** includes a phototherapy blanket **12** and a urine collector **14** that are each disposed on, or adjacent to, a neonate (or other person) **P**. The phototherapy blanket **12** at least partially encases the neonate **P**, and is preferably made of cloth or a textile chosen to provide desirable properties such as conformability to the body or enclosing comfort to the neonate **P**. As described herein, the phototherapy apparatus **10** provides phototherapy and additionally measures at least one target biomarker in urine excreted by the neonate **P**. The target biomarker is indicative of the neonate's liver function (i.e. normal bilirubin elimination) and/or is indicative of an adverse side effect of the phototherapy. In some embodiments, the at least one target biomarker includes at least one of isoprostane concentration in urine, and urobilin concentration in urine. Isoprostane concentration in urine is indicative of lipid peroxidation which may increase due to a combination of production of reactive oxygen species and reduction of bilirubin due to the phototherapy - this increase in lipid peroxidation is a possible undesirable side-effect of the hyperbilirubinemia phototherapy, and hence if the isoprostane concentration increases above some chosen threshold value this may be a basis for recommending reduction in the intensity or spatial extent of the phototherapy illumination, or for turning off the phototherapy illumination entirely. Urobilin accumulates in urine as a product of the normal breakdown of bilirubin, and hence a high urobilin concentration in urine indicates a liver that functions or is starting to function normally, while a low urobilin concentration in urine indicates continued liver deficiency in processing bilirubin and therefore (a probable) continued need for phototherapy.

With continuing reference to FIGURE 1, the phototherapy device **10** further includes an electronic processing device **16** comprising a microprocessor or microcontroller programmed to control the hyperbilirubinemia phototherapy delivered by at least one illumination device **20** of the phototherapy blanket **12**. In the illustrative example, the electronic processing device **16** is a phototherapy blanket controller. In the illustrative embodiment, the illuminators **20** are arranged on or in the phototherapy blanket **12** to illuminate the at least partially encased neonate **P** with phototherapy illumination effective to treat hyperbilirubinemia. Note that the illuminators **20** are arranged between an outer and inner surface of the blanket **12** so that the phototherapy light is emitted from a side of the blanket facing the neonate **P**, and accordingly the illuminators **20** are occluded from view by the blanket **12** and accordingly are shown using dashed lines to indicate the illuminators **20** are hidden features in the perspective view of FIGURE 1.

According to guidance from the American Academy of Pediatrics (AAP), phototherapy illumination recommended as effective to treat hyperbilirubinemia has emission in the blue-to-green spectrum, typically 460-490 nm. It will be appreciated that illumination having a wavelength range spanning only a portion of this range (e.g. 450-470 nm), or even emitting at a single wavelength in this range, and/or extending outside of this range (e.g. 465-520 nm), should also be effective. The AAP guidance further recommends irradiance of at least 30 microwatts/cm²/nm (for example, measured using an irradiance meter calibrated over the chosen wavelength range). It will again be appreciated that this is recommended guidance and that a lower irradiance may still provide therapeutic benefit, while higher irradiance is likely to provide increasing benefit, i.e. increasing rate of bilirubin breakdown albeit possibly with increased undesirable side-effects. The AAP guidance further recommends illuminating the neonate over a large a portion of the body surface as possible, and indicates that TSB reduction should be observed during the first 4 to 6 hours of exposure if the phototherapy is effective.

In one suitable embodiment, the illuminators **20** are light emitting diodes (LEDs) arranged on or in the phototherapy blanket **12** and configured to emit light in the wavelength range 460-490 nm. In one suitable assembly, the LEDs are mounted on textile ribbons with conductive wires attached in roughly parallel rows on the inner side of the outer surface of the phototherapy blanket **12** that is, on the side facing the neonate **P**. The ribbons provide a convenient way to mount the LEDs and to route electrical power to the LEDs, but alternative assemblies are contemplated, such as mounting LEDs on flexible circuit board strips or tabs sewn onto the blanket with discrete wires running through the fabric of the phototherapy blanket **12**. The phototherapy blanket may be a multi-layer blanket, for example with a spacer layer to maintain a distance between the LEDs and the skin of the neonate **P** (with apertures for the LEDs), and an inner liner to protect the neonate from contact with the illuminators and their wiring or circuit boards (such a liner may prevent touching of the LEDs through the apertures and ingression of dirt and fluids into the blanket, or may be thin enough and sufficiently translucent in the therapeutic wavelength range, e.g. 460-490 nm, to permit effective phototherapy). In other embodiments, the blanket **12** can include other types of light sources (e.g., vertical-cavity surface-emitting lasers (VCSELs) or light from an external source that may be guided to the blanket with optical fibers. Some suitable phototherapy blanket embodiments are described, by way of illustration, in Asvadi et al., Int'l. Pub. WO 2007/091188 A2.

The phototherapy blanket controller **16** preferably controls the illuminators **20** in order to control the therapeutic illumination output by the illuminators **20**. This control can be open-loop, e.g. the phototherapy blanket controller **16** may apply a fixed electrical current to the electrical circuit driving the illuminators **20**, or the control may be closed-loop using a feedback signal provided by one or more photodetectors (not shown) also arranged on or in the phototherapy blanket **12**. The illustrative controller **16** further includes a display component **22** via which the user (e.g. nurse, physician, parent, or so forth) is informed of the current phototherapy setting(s) or other settings (connection to terminals, battery status, and the like), and which has one or more user input buttons or the like **24** via which the user can set up the phototherapy or optionally perform other user interfacing operations.

Advantageously, the illustrative phototherapy device **12** having a blanket form at least partially encasing the neonate **P** provides illumination of the neonate **P** over most of the neonate's external surface area (thus maximizing effectiveness of the hyperbilirubinemia phototherapy according to the AAP guidance) while still providing for the neonate **P** to be clothed (in the phototherapy blanket **12**) and advantageously reducing or eliminating exposure of the neonate's eyes to the therapeutic illumination. In some embodiments, the controller **16** can be set to power only a sub-set of the illuminators **20**, for example only the illuminators on the front side of the neonate **P** (or, alternatively, only on the back side of the neonate, or alternatively every other strip may be turned off, et cetera). This provides for adjustment of the spatial distribution of the phototherapy illumination which in some cases may be a preferable way to control the hyperbilirubinemia phototherapy as compared with adjusting the illumination intensity (or both intensity and spatial area may be adjusted).

While the illustrative embodiment employs a hyperbilirubinemia phototherapy device in the form of the illustrative phototherapy blanket **12** including LED illuminators **20**, the disclosed approaches are also compatible with other types of hyperbilirubinemia phototherapy devices, such as an incubator with compact fluorescent lighting emitting at a therapeutic wavelength (e.g. in the 460-490 nm range) or a compact fluorescent or incandescent lamp arranged to similarly illuminate the neonate. These alternative approaches can be effective, but may have certain disadvantages such as illuminating the neonate over a smaller fraction of its total surface area, producing stray illumination into the neonate's eyes, requiring more electrical power, and requiring the neonate be naked so as to be exposed to the therapeutic illumination.

The urine collector **14** is positioned around the waist and/or buttocks of the neonate **P** so as to cover the genitalia (i.e. to collect urine). In some embodiments, the urine collector **14** is a conventional diaper; in other embodiments it is a diaper-like structure or absorbent pad; more generally, the urine collector **14** can have any configuration suitable for collecting (e.g. absorbing) urine. It is contemplated for the urine collector **14** to have different configurations for male versus female neonates. In an economical embodiment, the urine collector **14** is a disposable item, i.e. a diaper or other consumable, which is separate from the phototherapy blanket **12** which is arranged to surround the urine collector **14** (although as shown in FIGURE 1 the illuminators **20** typically do not overlap the urine collector **14** since the latter would block or absorb the therapeutic illumination, or, in some embodiments, the urine collector can include one or more non-transparent parts, such as an absorbing unit). Alternatively, the urine collector **14** can be detachably attached to the phototherapy blanket **12**, for example using Velcro™. The neonate **P** typically will urinate every 4-6 hours, and it is generally expected that a nurse, parent, or other caretaker will change out the urine collector **14** after each urination event.

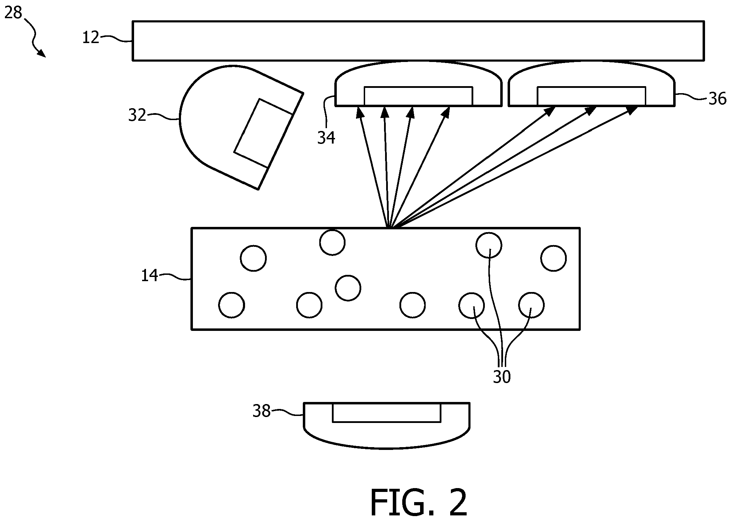

With continuing reference to FIGURE 1 and with further reference to FIGURE 2, as disclosed herein the hyperbilirubinemia phototherapy apparatus **10** provides feedback regarding liver function and/or side effects of the hyperbilirubinemia phototherapy via measurement of at least one target biomarker in urine excreted by the neonate **P**. To this end, at least one sensing device **28** is secured to the phototherapy blanket **12** and/or to the urine collector **14** and is configured to output a measurement of a target biomarker in urine collected by the urine collector **14**. In some embodiments, the sensing device **28** includes an immunoassay **30** (FIGURE 2), for example comprising an antibody that is embedded in or coated onto the urine collector, or a lateral flow assembly (not shown in FIGURE 2) and that exhibits a color change or a change in fluorescence in response to the concentration of a target biomarker to be detected in the collected urine exceeding some color transition threshold. Such a color change can be detected visually, e.g. observed by the nurse, parent, or other caregiver when changing out the diaper or other urine collector **14**. Additionally or alternatively, as seen in FIGURE 2 the sensing device **28** can include an optical detection sub-system for automatically detecting the color or fluorescence change. For example, in illustrative FIGURE 2 the sensing device **28** further includes a light source **32** secured to the phototherapy blanket **12** and at least one scattered light detector **34** or fluorescence detector **36** secured to the phototherapy blanket **12**, and/or a forward scattered light detector **38** secured to the urine collector **14** so as to detect light from the light source **32** that scatters through the urine collector **14** dependent on the color change of the urine sample. Advantageously, the use of an optical detection system **32**, **34**, **36**, **38** can provide more finely resolved data, e.g. not simply a binary "color change" or "no color change" assessment, but a quantitative measure of how much the color has changed, which can be a better method of judging whether the color change threshold has been passed.

Rather than embedding or coating the immunoassay **30** in or on the urine collector **14**, it is alternatively contemplated for the immunoassay to be a separate component, e.g. a cover sheet containing the immunoassay which is disposed over (and in intimate contact with) the urine collector. Some suitable target biomarkers, such as urobilin, produce a color change in the urine without the use of an immunoassay, and if such a biomarker is employed then the immunoassay may be omitted.

Various hyperbilirubinemia markers in urine can be detected in order to assess the liver function and/or in order to assess undesired side effects that may be geneated by the hyperbilirubinemia phototherapy. Some examples are given below.

In one approach, the detected target biomarker is isoprostane concentration in urine. This biomarker is related to the possible undesirable side effect of increased oxidative stress as follows. Phototherapy leads to the production of reactive oxygen species and the reduction of bilirubin, which is a potent antioxidant. The result can be an increased oxidative stress for the body of the infant, leading to lipid peroxidation. Isoprostanes in urine are a measure for lipid peroxidation. Isoprostanes can be suitably detected with the immunoassay **30** (biochemical test for detection of macromolecules based on coupling to antibodies and labelling) designed to produce a visibly detectable color change observable by the caretaker as a function of isoprostane concentration, and/or the color change may be detected with the optical sensor **34**, **36**, **38**. Rather than detecting a color change, the immunoassay **30** may be designed to produce a detectable fluorescence as the label, which is suitably produced in response to illumination by the light source **32** and detected by the fluorescence detector **36**. Although FIGURE 2 shows that the optical sensor **34** is adjacent to the light source **32**, it will be appreciated that the fluorescence detector **36** can be positioned adjacent to the light source.

In another approach, the detected target biomarker is urobilin, whose concentration in urine is related to the need of continued phototherapy as follows. When the neonate's liver starts to work normally it will remove (unconjugated) bilirubin from the blood eventually resulting in urobilin in the urine. As bilirubine is not soluable in water, it is coupled to albumine in the blood. A functioning liver takes it up and couples it to glucuron (by the enzyme glucuronyltransferase). Once coupled to glucuron, the bilirubine can be excreted in the bile. Via the gall bladder the conjugated bilirubine is excreted in the intestines. In the intestines the bilirubine is converted to urobilinogene. Urobilinogene is oxidized either in the intestines (to stercobiline) or taken up in the blood and then oxidized to urobilin in the kidneys. Urobilin gives the color to urine. The color of the urine in the urine collector **14** may be a direct indication of the liver function (in which case the immunoassay **30** is omitted), or the immunoassay **30** is provided for more accurate detection. A resulting color change or fluorescence is detected by the optical detection system **32**, **34**, **36**, **38**. Alternatively, urobilin can be detected via absorption spectrum analysis using the light source **32** and detector **38**. It will be appreciated that other biomarkers may detect efficacy of the phototherapy, such as a decrease of TSB.

Each of the optical components **32**, **34**, **36**, **38** of the sensing device **28**, if provided, may be secured to the phototherapy blanket **12** and/or to the urine collector **14**. Because the urine collector **14** becomes soiled each time the neonate **P** urinates (which occurs every 4-6 hours typically), the urine collector **14** is preferably a disposable item. As such, it may be more cost-effective to secure most or all of the optical components **32**, **34**, **36** to the phototherapy blanket **12** so that they are not replaced with each urine collector change-out. Alternatively, the urine collector may be washable/reusable, but in this case any attached optical components would need to be robust against the washing cycle. Optical components **32**, **34**, **36** which are mounted to the phototherapy blanket **12** should be positioned so as to interact appropriately with the urine collector **14**, e.g. the light source **32** should be arranged to apply light to the urine collector **14** in a region likely to have a large quantity of collected urine, and the optical sensors **34**, **36** should be positioned to detect that light after scattering or to detect induced fluorescence or other optical output generated by the label of the immunoassay **30**. In general, the optical detection can detect the labeled target biomarker by detecting forward-scatter of the cells, side scatter of the cells, fluorescent emission of the cells, or so forth. The relative amount of optical power on the detector **34**, **36**, **38** is measured. The light source **32** emits light in a wavelength range effective to activate the immunoassay label. In some embodiments, the excitation wavelength is in the ultraviolet to blue range (200-490 nm), e.g. between 200-290 nm or between 450-490 nm. For example, the light source **32** can be a collimated LED source, or alternatively, a laser source for small collimation angle and specific spectral excitation. Measuring fluorescence, side scattering and forward scattering in a flowing fluid is known as Laser Flow Cytometry. It will be noted that the latter wavelength range overlaps the AAP-recommended therapeutic wavelength range of 460-490 nm for hyperbilirubinemia phototherapy - if this is the case for the deployed immunoassay **30**, then it is contemplated to employ the illuminators **20** as the light source for optically activating the immunoassay label (so that the separate activation light source **32** is optionally omitted).

During operation, the sensing device **28** measures at least one target biomarker in urine collected by the urine collector **14**. If the sensing device **28** is an immunoassay that exhibits a color change intended to be detected visually by the caregiver during change-out of the urine collector **14**, then the optical components **32**, **34**, **36**, **38** are suitably omitted and detection of the target biomarker is performed manually by visual inspection of the urine collector **14**.

With returning reference to FIGURE 1, if on the other hand optical sensing components **32**, **34**, **36**, **38** are employed, these are preferably connected with the phototherapy blanket controller **16** (or some other electronic data processing device) by suitable wiring **40** or by a wireless connection, and the controller **16** (or more particularly an electronic processor of the controller) is programmed to generate a hyperbilirubinemia phototherapy recommendation, for example selected from a group consisting of: (i) a recommendation to perform a TSB test; (ii) a recommendation to continue phototherapy illumination, (iii) a recommendation to adjust intensity of the phototherapy illumination, (iv) a recommendation to adjust spatial distribution of the phototherapy illumination, and (v) a recommendation to turn off the phototherapy illumination.

In some embodiments, a TSB test is to be performed by the caregiver. Based on the outcome of this test, the physician responsible for the neonate decides whether to reduce or discontinue the phototherapy. The provided information on the concentration of biomarkers for side-effects or normal liver function can be taken into account in this decision. It will be appreciated that the TSB test provides a confirmation as to whether to reduce or discontinue phototherapy, as a TSB test is considered the "gold standard" in determining whether to apply photohterapy. However, measurement of the target biomarkers (e.g., isoprostane, urobilin, and the like) can also be used to determine whether to begin/reduce/discontinue phototherapy.

As another example, if the target biomarker is isoprostane concentration in urine, then the controller **16** is programmed to generate a recommendation to reduce intensity (i.e. average spectral irradiance) or spatial distribution (i.e. body coverage is reduced while the spectral irradiance in the illuminated parts remains the same) of the phototherapy illumination or to turn off the phototherapy illumination entirely if the measurement of isoprostane concentration in urine is greater than a threshold. Such recommendations are motivated by high isoprostane concentration being indicative of the neonate **P** experiencing increased oxidative stress due to the hyperbilirubinemia phototherapy.

As yet another example, if the target biomarker is urobilin concentration in urine, then the controller **16** is programmed to generate a recommendation to generate a hyperbilirubinemia phototherapy recommendation indicating starting or normal liver functionality (independent of phototherapy) of the neonate being treated by the phototherapy device **12**, or, alternatively, a reduction of intensity or spatial coverage, or turning of the illuminators **20**. In this example, a higher urobilin concentration in urine is indicative of normal elimination of bilirubin by the liver of the neonate. Therefore phototherapy may be reduced or discontinued.

The hyperbilirubinemia phototherapy recommendation may be used in various ways. In one approach, the controller **16** directly controls the phototherapy device (e.g. the illuminators **20** of the phototherapy blanket **12** in the illustrative embodiment) to implement the hyperbilirubinemia phototherapy recommendation, e.g. by switching off some or all of the illuminators **20**, and/or reducing their drive current, in response to the isoprostane concentration in urine exceeding some threshold value chosen as indicative of excessive oxidative stress.

However, directly controlling the phototherapy on the basis of the generated hyperbilirubinemia phototherapy recommendation may be problematic. Such control may contravene therapy prescribed by the neonate's physician. Additionally, target biomarkers in the urine are generally considered less reliable than the "gold standard" of total serum bilirubin (TSB) measured via a blood test.

Accordingly, in some embodiments the hyperbilirubinemia phototherapy recommendation is not directly implemented by the phototherapy controller **16**, but rather is communicated to the nurse or caregiver, for example via the display component **22** of the controller **16**. Additionally or alternatively, the controller **16** may be provided with a flashing light, audio alarm or the like which may be activated based on the hyperbilirubinemia phototherapy recommendation. Additionally or alternatively, the hyperbilirubinemia phototherapy recommendation may be communicated to a neonatal ward nurses' station or the like via a wired or wireless communication pathway.

The measurement of the monitored target biomarker in urine generated by the sensing device **28** is valid only when the urine collector **14** has collected a sufficient quantity of urine. Typically, a neonate urinates every four to six hours, and the urine collector **14** is changed out shortly thereafter. Accordingly, the measurement output by the sensing device **28** is valid for the time interval between the urination event and the change-out. Typically, the output of the optical detector **34**, **36**, **38** will have a readily identified "no signal" output in the time interval before urine is collected. For example, the luminescence detector **36** will output a low or null signal until the biomarker-containing urine is excreted, while a transmission detector may have a high signal until a light-absorbing biomarker in urine is present. The optical detection system may measure at different wavelengths of emission of the light source 32 or different wavelengths of sensitivity of detectors 34, 38, or a combination of color and fluorescence measurements to detect presence of urine on the one hand (independent of biomarker concentration) and biomarker quantity on the other hand.

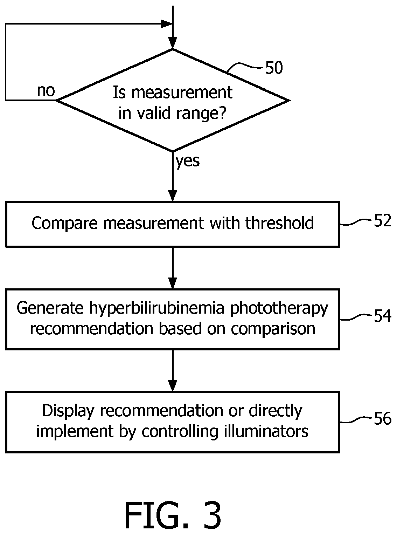

With reference to FIGURE 3, a suitable processing sequence in view of the foregoing is described. In an operation **50**, the sensing device **28** continually generates measurements, which are checked for validity. Until the neonate **P** urinates, these validity checks will indicate invalid measurements. When a valid measurement is detected at operation **50**, thus indicating the neonate **P** has urinated and the urine has collected in the urine collector **14**, processing flows to an operation **52** which compares the measurement with a threshold, and then to an operation **54** at which the hyperbilirubinemia phototherapy recommendation is generated based on this threshold. In an operation **56**, the hyperbilirubinemia phototherapy recommendation is displayed (or alternatively the phototherapy apparatus is directly controlled to implement the hyperbilirubinemia phototherapy recommendation).

In the illustrative examples, the subject is a neonate **P** having neonatal hyperbilirubinemia. However, hyperbilirubinemia can also afflict adults, e.g. elderly patients with a jaundice condition, Crigler-Najjar syndrome, and the like, and the disclosed hyperbilirubinemia phototherapy apparatuses and methods are readily adapted for such an adult patient (e.g. by substituting an adult-sized phototherapy blanket for the illustrative neonatal therapy blanket **12**).

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. A hyperbilirubinemia phototherapy apparatus (**10**) comprising
a phototherapy device (**12**) including illuminators (**20**) arranged to illuminate a neonate with phototherapy illumination effective to treat hyperbilirubinemia; **characterized by**
a sensing device (**28**) secured to the phototherapy device and configured to output a measurement of a target biomarker in urine excreted by a neonate receiving phototherapy illumination from the phototherapy device.

2. The hyperbilirubinemia phototherapy apparatus of claim 1 wherein:
the phototherapy device comprises a phototherapy blanket (**12**) configured to at least partially encase a neonate, the phototherapy blanket including the illuminators (**20**) arranged on or in the phototherapy blanket to illuminate the at least partially encased neonate with phototherapy illumination effective to treat hyperbilirubinemia.

3. The hyperbilirubinemia phototherapy apparatus of claim 2 wherein the sensing device (**28**) includes:
a light source (**32**) secured to the phototherapy blanket (**12**) and configured to emit light onto a diaper or other urine collector (**14**); and
a light detector (**34**, **36**, **38**) secured to the phototherapy blanket (**12**) and configured to detect at least one of scattered, reflected, transmitted, and fluorescent light emanating from the diaper or other urine collector responsive to light emitted onto the diaper or other urine collector by the light source.

4. The hyperbilirubinemia phototherapy apparatus of any one of claims 2-3 wherein the illuminators (**20**) comprise light emitting diodes (LEDs) arranged on or in the phototherapy blanket (**12**) and configured to emit light in the wavelength range 460-490 nm.

5. The hyperbilirubinemia phototherapy apparatus of any one of claims 2-4 further comprising:
a phototherapy blanket controller (**16**) configured to control the illuminators (**20**) of the phototherapy blanket (**12**) and to display a hyperbilirubinemia phototherapy recommendation generated by the phototherapy blanket controller from a measurement of the target biomarker output by the sensing device (**28**).

6. The hyperbilirubinemia phototherapy apparatus of claim 5 wherein the phototherapy blanket controller (**16**) is programmed to generate the hyperbilirubinemia phototherapy recommendation selected from a group consisting of: (i) a recommendation to perform a TSB test; (ii) a recommendation to continue phototherapy illumination, (iii) a recommendation to adjust intensity of the phototherapy illumination, (iv) a recommendation to adjust spatial distribution of the phototherapy illumination, and (v) a recommendation to turn off the phototherapy illumination.

7. The hyperbilirubinemia phototherapy apparatus of claim 6 wherein the sensing device (**28**) is configured to output a measurement of isoprostane concentration in urine and the phototherapy blanket controller (**16**) is programmed to generate a recommendation to reduce intensity or spatial distribution of the phototherapy illumination or to turn off the phototherapy illumination if the measurement of isoprostane concentration in urine is greater than a threshold.

8. The hyperbilirubinemia phototherapy apparatus of claim 6 wherein the sensing device (**28**) is configured to output a measurement of urobilin concentration in urine and the phototherapy blanket controller (**16**) is programmed to generate a hyperbilirubinemia phototherapy recommendation indicating normal liver functionality of the neonate being treated by the phototherapy device (**12**) based on the measurement of urobilin concentration in urine.

9. The hyperbilirubinemia phototherapy apparatus of any one of claims 5-8 wherein the phototherapy blanket controller (**16**) is attached to the phototherapy device directly or by an electrical cord (**40**).

10. The hyperbilirubinemia phototherapy apparatus of any one of claims 1-9 wherein the sensing device (**28**) includes at least one immunoassay configured to output a signal indicative of a target biomarker concentration in urine, the signal including a change in urine color based on the target biomarker concentration.

11. The hyperbilirubinemia phototherapy apparatus of any one of claims 1-10 wherein the sensing device (**28**) is configured to output a measurement of a target biomarker comprising at least one of isoprostane concentration in urine and urobilin concentration in urine.

12. The hyperbilirubinemia phototherapy apparatus of any one of claims 1-11 further comprising a urine collector (14) configured to collect urine excreted by the neonate while being illuminated with the phototherapy illumination by the phototherapy device.

## Patentansprüche

1. Hyperbilirubinämie-Phototherapiegerät (10) umfassend
eine Phototherapievorrichtung (12) einschließlich Illuminatoren (20), die dafür ausgelegt sind, ein Neugeborenes mit Phototherapielicht zu beleuchten, das wirksam zur Behandlung von Hyperbilirubinämie ist, **gekennzeichnet durch**
eine Erfassungsvorrichtung (28), die an der Phototherapievorrichtung befestigt ist und konfiguriert ist, um eine Messung eines Ziel-Biomarkers in Urin auszugeben, der durch ein Neugeborenes ausgeschieden wird, das Phototherapielicht aus der Phototherapievorrichtung empfängt.

2. Hyperbilirubinämie-Phototherapiegerät nach Anspruch 1, wobei
die Phototherapievorrichtung eine Phototherapiedecke (12) umfasst, die konfiguriert ist, um ein Neugeborenes zumindest teilweise einzuhüllen, wobei die Phototherapiedecke die Illuminatoren (20) einschließt, welche an oder in der Phototherapiedecke angeordnet sind, um das zumindest teilweise eingehüllte Neugeborene mit Phototherapielicht zu beleuchten, das wirksam zur Behandlung von Hyperbilirubinämie ist.

3. Hyperbilirubinämie-Phototherapiegerät nach Anspruch 2, wobei die Erfassungsvorrichtung (28) Folgendes umfasst:
eine Lichtquelle (32), die an der Phototherapiedecke (12) befestigt ist und dafür konfiguriert ist, Licht auf eine Winkel oder einen anderen Urinfänger (14) zu emittieren; und
einen Lichtdetektor (34, 36, 38), der an der Phototherapiedecke (12) befestigt ist und dafür konfiguriert ist, mindestens eines von gestreutem, reflektiertem, übertragenem und fluoreszierendem Licht zu detektieren, das aus der Windel oder dem anderen Urinfänger austritt, wenn Licht durch die Lichtquelle auf die Windel oder den anderen Urinfänger emittiert wird.

4. Hyperbilirubinämie-Phototherapiegerät nach einem der Ansprüche 2 bis 3, wobei die Illuminatoren (20) Leuchtdioden (LEDs) umfassen, die auf oder in der Phototherapiedecke (12) angeordnet sind und konfiguriert sind, um Licht im Wellenlängenbereich von 460 bis 490 nm zu emittieren.

5. Hyperbilirubinämie-Phototherapiegerät nach einem der Ansprüche 2 bis 4, ferner umfassend:
eine Phototherapiedecken-Steuereinheit (16), die konfiguriert ist, um die Illuminatoren (20) der Phototherapiedecke (12) zu steuern und eine Hyperbilirubinämie-Phototherapieempfehlung anzuzeigen, die durch die Phototherapiedecken-Steuereinheit anhand einer Messung des Ziel-Biomarkers erzeugt wird, welche durch die Erfassungsvorrichtung (28) ausgegeben wird.

6. Hyperbilirubinämie-Phototherapiegerät nach Anspruch 5, wobei die Phototherapiedecken-Steuereinheit (16) programmiert ist, um die Hyperbilirubinämie-Phototherapieempfehlung ausgewählt aus einer Gruppe zu erzeugen, die besteht aus: (i) einer Empfehlung zur Durchführung eines TSB-Tests; (ii) einer Empfehlung zur Fortsetzung der Phototherapie-Beleuchtung, (iii) einer Empfehlung zum Anpassen der Intensität der Phototherapie-Beleuchtung, (iv) einer Empfehlung zum Anpassen der räumlichen Verteilung der Phototherapie-Beleuchtung, und (v) einer Empfehlung zum Ausschalten der Phototherapie-Beleuchtung.

7. Hyperbilirubinämie-Phototherapiegerät nach Anspruch 6, wobei die Erfassungsvorrichtung (28) konfiguriert ist, um eine Messung der Isoprostan-Konzentration im Urin auszugeben, und wobei die Phototherapiedecken-Steuereinheit (16) programmiert ist, um eine Empfehlung zur Reduzierung der Intensität oder der räumlichen Verteilung der Phototherapie-Beleuchtung oder zum Ausschalten der Phototherapie-Beleuchtung zu erzeugen, wenn die gemessene Isoprostan-Konzentration im Urin höher als ein Schwellenwert ist.

8. Hyperbilirubinämie-Phototherapiegerät nach Anspruch 6, wobei die Erfassungsvorrichtung (28) konfiguriert ist, um eine Messung der Urobilin-Konzentration im Urin auszugeben, und wobei die Phototherapiedecken-Steuereinheit (16) programmiert ist, um eine Hyperbilirubinämie-Phototherapieempfehlung zu erzeugen, die basierend auf der Messung der Urobilin-Konzentration im Urin eine normale Leberfunktion des Neugeborenen angibt, das mit der Phototherapievorrichtung (12) behandelt wird.

9. Hyperbilirubinämie-Phototherapiegerät nach einem der Ansprüche 5 bis 8, wobei die Phototherapiedecken-Steuereinheit (16) direkt oder über ein elektrisches Kabel (40) an der Phototherapievorrichtung befestigt ist.

10. Hyperbilirubinämie-Phototherapiegerät nach einem der Ansprüche 1 bis 9, wobei die Erfassungsvorrichtung (28) mindestens ein Immunoassay umfasst, das konfiguriert ist, um ein Signal auszugeben, das eine Ziel-Biomarker-Konzentration im Urin angibt, wobei das Signal eine Änderung der Urinfarbe basierend auf der Ziel-Biomarker-Konzentration einschließt.

11. Hyperbilirubinämie-Phototherapiegerät nach einem der Ansprüche 1 bis 10, wobei die Erfassungsvorrichtung (28) konfiguriert ist, um eine Messung eines Ziel-Biomarkers auszugeben, die mindestens eine von der Isoprostan-Konzentration im Urin und der Urobilin-Konzentration im Urin umfasst.

12. Hyperbilirubinämie-Phototherapiegerät nach einem der Ansprüche 1 bis 11, ferner umfassend einen Urinfänger (14), der konfiguriert ist, um durch das Neugeborene ausgeschiedenen Urin aufzufangen, während es mit der Phototherapie-Beleuchtung durch die Phototherapievorrichtung beleuchtet wird.

## Revendications

1. Appareil de photothérapie contre l'hyperbilirubinémie (10) comprenant
un dispositif de photothérapie (12) incluant des dispositifs d'éclairage (20) agencés pour éclairer un nouveau-né avec un éclairage de photothérapie efficace pour traiter l'hyperbilirubinémie ; **caractérisé par**
un dispositif de détection (28) fixé au dispositif de photothérapie et configuré pour sortir une mesure d'un marqueur biologique cible dans l'urine excrétée par un nouveau-né recevant un éclairage de photothérapie en provenance du dispositif de photothérapie.

2. Appareil de photothérapie contre l'hyperbilirubinémie selon la revendication 1, dans lequel :
le dispositif de photothérapie comprend une couverture de photothérapie (12) configurée pour envelopper au moins partiellement un nouveau-né, la couverture de photothérapie incluant les dispositifs d'éclairage (20) agencés sur ou dans la couverture de photothérapie pour éclairer le nouveau-né au moins partiellement enveloppé avec l'éclairage de photothérapie efficace pour traiter l'hyperbilirubinémie.

3. Appareil de photothérapie contre l'hyperbilirubinémie selon la revendication 2, dans lequel le dispositif de détection (28) inclut :
une source de lumière (32) fixée à la couverture de photothérapie (12) et configurée pour émettre de la lumière sur une couche ou autre collecteur d'urine (14) ; et
un détecteur de lumière (34, 36, 38) fixé à la couverture de photothérapie (12) et configuré pour détecter au moins l'une d'une lumière dispersée, réfléchie, transmise et fluorescente émanant de la couche ou autre collecteur d'urine en réponse à la lumière émise sur la couche ou autre collecteur d'urine par la source de lumière.

4. Appareil de photothérapie contre l'hyperbilirubinémie selon l'une quelconque des revendications 2 à 3, dans lequel les dispositifs d'éclairage (20) comprennent des diodes électroluminescentes (LED) agencées sur ou dans la couverture de photothérapie (12) et configurés pour émettre de la lumière dans la plage de longueurs d'onde de 460 à 490 nm.

5. Appareil de photothérapie contre l'hyperbilirubinémie selon l'une quelconque des revendications 2 à 4 comprenant en outre :
une unité de commande de couverture de photothérapie (16) configurée pour commander les dispositifs d'éclairage (20) de la couverture de photothérapie (12) et pour afficher une recommandation de photothérapie contre l'hyperbilirubinémie produite par l'unité de commande de couverture de photothérapie à partir d'une mesure de la sortie de marqueur biologique cible par le dispositif de détection (28).

6. Appareil de photothérapie contre l'hyperbilirubinémie selon la revendication 5, dans lequel l'unité de commande de couverture de photothérapie (16) est programmée pour produire la recommandation de photothérapie contre l'hyperbilirubinémie sélectionnée dans un groupe constitué par : (i) une recommandation pour effectuer un test de TSB ; (ii) une recommandation pour continuer l'éclairage de photothérapie, (iii) une recommandation pour ajuster l'intensité de l'éclairage de photothérapie, (iv) une recommandation pour ajuster la distribution spatiale de l'éclairage de photothérapie et (v) une recommandation pour éteindre l'éclairage de photothérapie.

7. Appareil de photothérapie contre l'hyperbilirubinémie selon la revendication 6, dans lequel le dispositif de détection (28) est configuré pour sortir une mesure de concentration en isoprostane dans l'urine et l'unité de commande de couverture de photothérapie (16) est programmée pour produire une recommandation pour réduire l'intensité ou la distribution spatiale de l'éclairage de photothérapie ou pour éteindre l'éclairage de photothérapie si la mesure de concentration en isoprostane dans l'urine est plus grande qu'un seuil.

8. Appareil de photothérapie contre l'hyperbilirubinémie selon la revendication 6, dans lequel le dispositif de détection (28) est configuré pour sortir une mesure de concentration en urobiline dans l'urine et l'unité de commande de couverture de photothérapie (16) est programmée pour produire une recommandation de photothérapie contre l'hyperbilirubinémie indiquant une fonctionnalité de foie normale du nouveau-né traité par le dispositif de photothérapie (12) sur la base de la mesure de concentration en urobiline dans l'urine.

9. Appareil de photothérapie contre l'hyperbilirubinémie selon l'une quelconque des revendications 5 à 8, dans lequel l'unité de commande de couverture de photothérapie (16) est attachée au dispositif de photothérapie directement ou par un cordon électrique (40).

10. Appareil de photothérapie contre l'hyperbilirubinémie selon l'une quelconque des revendications 1 à 9, dans lequel le dispositif de détection (28) inclut au moins un immunoessai configuré pour sortir un signal indicatif d'une concentration de marqueur biologique cible dans l'urine, le signal incluant un changement de la couleur de l'urine sur la base de la concentration en marqueur biologique cible.

11. Appareil de photothérapie contre l'hyperbilirubinémie selon l'une quelconque des revendications 1 à 10, dans lequel le dispositif de détection (28) est configuré pour sortir une mesure d'un marqueur biologique cible comprenant au moins l'une de concentration en isoprostane dans l'urine et de concentration en urobiline dans l'urine.

12. Appareil de photothérapie contre l'hyperbilirubinémie selon l'une quelconque des revendications 1 à 11, comprenant en outre un collecteur d'urine (14) configuré pour collecter l'urine excrétée par le nouveau-né tout en étant éclairé par l'éclairage de photothérapie par le dispositif de photothérapie.
